Europäisches Patentamt

European Patent Office

Office européen des brevets

(11) Publication number: **0 245 056**
**A1**

## (12) EUROPEAN PATENT APPLICATION

(21) Application number: **87303958.0**

(22) Date of filing: **01.05.87**

(51) Int. Cl.4: **A 61 M 5/14**

(30) Priority: **07.05.86 US 860581**

(43) Date of publication of application:
**11.11.87 Bulletin 87/46**

(84) Designated Contracting States:
**AT BE CH DE ES FR GB GR IT LI LU NL SE**

(71) Applicant: **Vaillancourt, Vincent L.**
**VLV Associates Inc. 30A Ridgedale Avenue**
**East Hanover New Jersey 07939 (US)**

(72) Inventor: **Vaillancourt, Vincent L.**
**VLV Associates Inc. 30A Ridgedale Avenue**
**East Hanover New Jersey 07939 (US)**

(74) Representative: **Silverman, Warren et al**
**HASELTINE LAKE & CO. Hazlitt House 28 Southampton**
**Buildings Chancery Lane**
**London WC2A 1AT (GB)**

(54) Ambulatory disposable infusion pump.

(57) An ambulatory disposable infusion pump has a preferably substantially transparent receiving and retaining housing (20) which may have indicia markings (23) conventionally applied to indicate quantity. The inflow of fluid causes an elastomeric member (56) attached to a piston (28) which is slideable in the housing to be stretched. The tension in the elastomeric member provides an energy source (force) to push the fluid out of the bore when the connected tubing line (40) is opened.

Fig. 1

**Description**

AMBULATORY DISPOSABLE INFUSION PUMP

This invention relates to an ambulatory infusion pump and, more particularly, relates to an improvement in a disposable infusion delivery system.

Many infusion systems have been proposed and sold for the dispensing of drugs into a patient. With few exceptions, these systems contain both reusable (hardware components) and disposable parts. In some cases, these systems include, for the most part, electrical components, with their attendant costs and limitations.

As is known, the delivery of medication may include a catheter as a vascular access device or dispensing may be through a needle penetrating an implanted vascular access port which, in turn is connected to a blood vessel using an internal catheter. The delivery of this medication usually requires a small receiving means with delivery through a catheter-vascular access port at a constant rate for an extended and determined period of time. Devices of this type are preferably light in weight and pocket accessible. The devices are normally disposed of at completion of the infused dosage of medication for convenience, elimination of mixed drugs, and minimizing contamination. At present, most, if not all, disposable ambulatory medication or drug infusion devices employ an elastomeric bladder which is caused to be expanded as a drug is fed or otherwise flows into the bladder. Subsequent delivery of the drug is dependent upon the capability of the bladder to expand and contract. U.S. Patent 3,486,539 describes a bladder as a means of storage and contraction to provide expulsion while providing pressure and filling a bladder through a front-connected needle with a flow metering restrictor. U.S. Patent 3,993,069 shows an expansible elastomeric bladder with a separate flow-control device and an outer flow restrictor. U.S. Patent 4,318,400 describes an elastomeric bladder-powered infuser and a tubular case enclosure to prevent excessive expansion of the bladder. Drugs are fed through a needle and syringe to the bladder through a resilient plug at the rear of the device. U.S. Patent 4,419,096 describes an apparatus very much like those noted above but with an added lumen and bulbar filler portions to establish the contracted shape of the elastomeric bladder when the contents are, or substantially are, expelled.

Briefly, the invention provides an ambulatory infusion pump which includes a housing having an inlet aperture for passage of a drug or other fluid medication into the housing, an outlet aperture for passage of the received drug from the housing and a piston which is slidably mounted in the housing. The pump also includes an elastomeric member such as a rubber tube which is secured between and to the housing and piston to stretch in response to movement of the piston during filling of the housing and to impose a constant force on the piston after filling for expelling the drug through the outlet aperture which, for this purpose, is a restrictor for controlling the rate of drug flow from the housing.

The infusion pump is a small, lightweight cylindrical apparatus which can be filled with fluid through a delivery device such as a hypodermic needle attached to a syringe and/or a pressurized container. The inflow of fluid causes the slideable piston to move rearwardly in the housing to elongate the elastomeric member. In this manner, energy is stored which is subsequently used to deliver this same fluid to an attached connector device to a patient's previously inserted catheter. This fluid is delivered at a predetermined rate of flow governed by the force provided by the stretched resilient member. This rate is thus predictable with a determined restrictor size.

The infusion pump may be sized to be carried inconspicuously on the patient's body and can be used as a disposable product so contamination, cleaning and resterilization problems are not present. The flow of stored fluid is self-regulated without constant monitoring. The fluid can be observed to determine volume remaining or released, clarity of drug (contamination not present).

The infusion pump is easily made and assembled. The interface between the elastomeric member and drug is minimal when compared to above-known bladder-type devices, thereby reducing the potential for extractables. The interior chamber between the piston and the end wall, unlike bladder products, can and does go toward a zero volume since the elastomeric member is preloaded (tensioned). The barrel in which the drug is carried can be made transparent so that drugs may be, and usually are, observed prior to and during infusion. This visual potential allows the user to detect contamination, color changes, etc. The leading edge of the piston may be used as a measuring means to measure accurately the quantity of medication infused. The delivery tubing conventionally has a connector by which a disconnect is achieved with a minimum effort, permitting replacement of the device after a one-time use. The tubing from the device usually includes a luer lock hub connector which may include a filter and flow-control restrictor.

In use, the fluid to be injected is flowed into the housing through a one-way valve and/or injection port. The inflow of fluid under pressure into the housing causes the piston to move rearwardly as this fluid fills the housing. The rubber tube that supplies the compressing force is stretched (placed under tension) until the housing is filled with the desired amount. The injection port is then closed. The device is primed by cracking an outlet female luer adapter, with the storage unit height being lower than the outlet. This device is now connected to the patient for a constant infusion delivery.

The infusion pump is made for a patient to wear inconspicuously (usually under clothing). The pump is silent in use and, except for the connection to the catheter in the patient, is entirely self-contained. The intravenous feeding is at a controlled rate, with no adjustment required, and permits this pump to be used in and with out-patient care. The administration of fluid through this infusion pump is continuous and rate-controlled, so a steady flow is provided to the patient. The delivery rate of infusion fluid is dependent upon the force developed by the stretched elastomeric member which is in tension in combination with the restrictor size. These two parameters determine the fluid delivery rate.

It is anticipated that the pump or system will hold about two to sixty cc.s of drug concentrate or mix, and as a fluid be dispensed through the connected tubing to a catheter/vascular access means in the patient. This delivery is a a constant rate for a period of time. The patient is not restricted but may be ambulatory. Also, the patient may visually ascertain when the fluid has been delivered, how much has been delivered as an infused dosage, and, when desired or empty, disconnected and discarded. A new-pump may then be connected to again provide a desired constant flow infusion.

The infusion pump is particularly useful for fluids used in chemotherapy, diabetes, pain relief and other drugs which exhibit a therapeutic effect through constant rate infusion. This pump also may be used as a replacement for syringe pumps for home therapy wherein the need for such pumps is indicated. This system of pump obviates the need for training and manipulating such pumps and is essentially pre-assembled, requires minimal training, no technical skills, and is self-regulating.

For a better understanding of the invention and to show how the same can be carried into effect, reference will now be made by way of example only to the accompanying drawings, wherein:

FIG. I represents a side view, partly in section and partly diagrammatic of one embodiment of a disposable infusion pump in accordance with the invention;

FIG. 2 represents an enlarged sectional side view of a forward extent of resilient tubing and a plug retainer for one end of the pump of Fig. I;

FIG. 3 represents an enlarged sectional side view showing the tubing of Fig. I with an end secured in and to a slideable piston;

FIG. 4 represents an enlarged fragmentary sectional side view showing a resilient plug mounted in an outlet aperture of the housing of Fig. I;

FIG. 5 represents an enlarged, and partly diagrammatic, sectional side view of luer lock connecting members;

FIGS. 6 A, 6 B and 6 C represent sectional side views of an alternate infusion pump with a slideable piston illustrated in three positions;

Fig. 7 represents an exploded view of the piston of Figs. 6A and a means of securing the end of a resilient tubing to the piston; and

Fig. 8 represents a sectional side view showing the piston of Fig. 7 in an assembled condition.

In the following description corresponding reference characters refer to like members throughout the several figures of the drawings.

Referring to Fig I, the infusion pump has an rigid cylindrical housing which is molded of a transparent or translucent plastic to define a barrel 2I with a relatively constant bore or diameter 22. This housing 20 also includes indicia 23 to indicate the fluid volume. These indicia may be molding rings, applied painted lines and/or numbers that are conventionally provided with syringes and like housings. A precise showing of graduations of indicia 23 differs with each device, as volume and desired flow rate are particular with each such device. For example, insulin has many differing dosages, etc., and the syringes used therewith have differing indicia. The rear or open end of barrel 2I is closed by a cap having a skirt 25 that is a snug fit within the bore 22. A vent or small aperture 26 is formed in and through this cap to allow air to enter the interior of the barrel 2I and prevent development of negative pressure as a piston 28 within the housing 20 is slid back and forth.

The piston 28 has a core 29 of substantially stiff material, such as polypropylene plastic, which retains its shape when in use and a tire portion 30 with right and left ribs or rings 3I, 32 which are shaped and sized to provide a minimum of friction with the interior bore 22 of the barrel 2I. These rings may be of Teflon (TM duPont) or polypropylene coated rubber, silicone rubber, coated neoprene and the like, or may have a treated surface to provide minimum friction while providing a seal when and while sliding. The forward face portion of this piston 28 is of spherical shape 33 to match a sperical configuration 34 at the front of the housing 20. The forward (left) end portion of the housing 20 is molded so as to have two projecting tubular portions 35,54. The outer portion 35 has a tapered shell 36 with a closed end 37 in which is formed a circular recess 38 for the insertion and retention of a flexible tubing 40. A small aperture 42 is formed in and through the closed end 37 to provide fluid flow capability or control and provides a fluid flow path from the interior of the outer protion 35 to the interior of the tubing 40.

The outer tapered shell 36 is molded or formed with a small communicating hole or inlet aperture 46 and, for the convenience of mounting and positioning, a collar portion 48 is also formed as this tapered portion 36 is molded. A resilient plug 50 is mounted in this collar portion 48 and aperture 46 to provide a resilient seal which may be pierced by a needle from a syringe (not shown).

The inner and shorter tubular member 54 which may be integral with the end wall of the barrel 2I is tubular in configuration and has an outward (left) end formed with a taper to receive and retain a short length of an elastomeric member in the form of a rubber tubing 56. This tubing 56 is secured in the tubular member 54 by a resilient tapered plug 58. The taper of the plug 58 is such that the force applied and tending to disclose the secured stretched tubing 56 tends to more securely retain the stretched tubing 56.

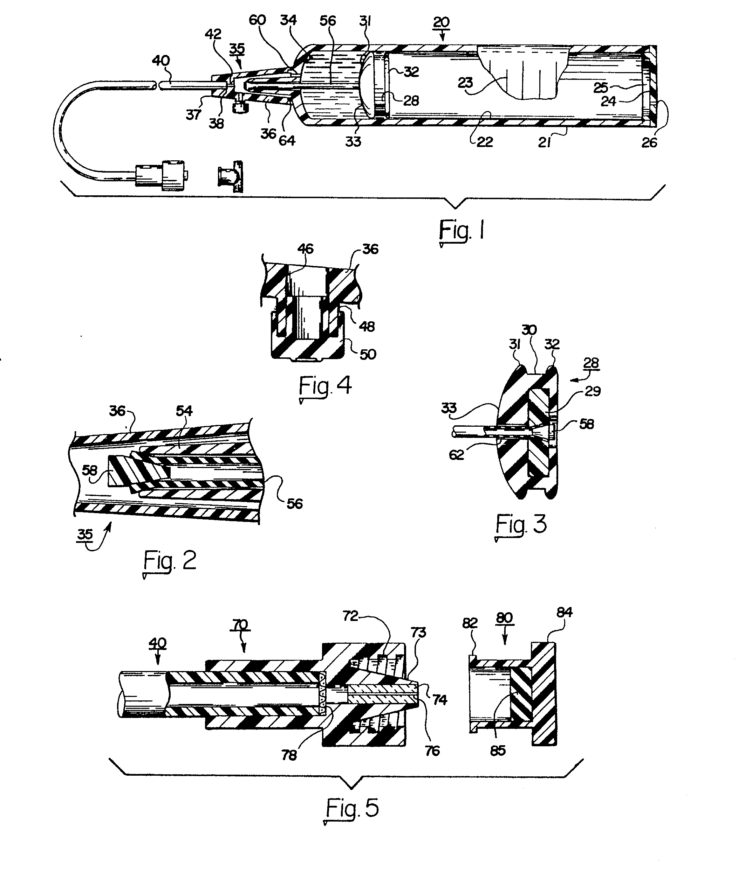

It is to be noted that in the integral wall portion between the tapered shell 36 and the tubular member 54 is a plurality of through holes 60. These holes provide fluid pathways from the interior of the housing 20 and the cavity between the shell 36 and the member 54. The piston 28 is likewise provided with a through hole or aperture 62 as shown in Fig. 3 sized and adapted to receive the other end of tubing 56, with another and like plug 58 securing this tubing 54 in the desired stretched condition.

For ease of manufacture and assembly, the tapered shell 36 is molded as a separate member and, as shown, has a shouldered joining 64 to provide a positive seating and securement. Sonic welding, cement or like means may be used so as to make the shell 36 fluid-tight with the housing 20 and with the desired strength capability.

In Fig. 5 there is depicted a luer lock device for connecting and disconnecting the flexible tubing 40 to a catheter (not shown) mounted in a patient to deliver a drug in a controlled amount. As shown, a molded luer connection member 70 has threads 72 formed therein and a male tapered central portion 73 with a mounted fluid restrictor 74 which has a predetermined through aperture 76. To the left and at the end of secured tube 40 is a bacteria filter disc 78. A mating winged luer cap connector 80 has an eared portion 82 and a winged portion 84 providing manipulating means. This eared portion 82 is adapted to engage the threaded portion 72 and, when winged portion 84 is rotated, the male portion 73 is drawn into the connector 80 to close the aperture 76. The winged cap may be of rubber which is sufficiently resilient to act as or provide a resilient closure. A softer resilient member 85 may be mounted in the cavity to provide and insure that the connector 70 is closed to a flow of fluid through conductor 40. The connector 70 and winged cap 80 utilized therewith are known.

The infusion pump may be constructed in an alternate manner for high-speed automatic assembly. In this alternate embodiment, a molded housing 90 is formed with a barrel 91 of a substantially constant diameter 92. The rear end of this molding may be closed by cap 94 having a skirt portion 95 that is a snug fit withinthe bore 92. A vent or aperture 96 is also provided in the cap 94 which allows atmospheric air to enter enter or air within the barrel to be expelled so that development of negative or positive pressure within the housing 90 does not occur when and with a sliding motion of a piston 98. This housing 90 is formed with a forward wall 100 in which there are formed three through apertures 102, 104, 106. The substantially central central aperture 102 is provided with an outwardly-extending collar portion having an internal configuration with a taper. The second aperture 104 is also formed with an outwardly-extending collar portion with a stepped bore and with a larger exterior bore sized to accept and retain the end of tubing 40. The other aperture 106 is sized to accept and retain a one-way valve (not shown) or a resilient plug 50.

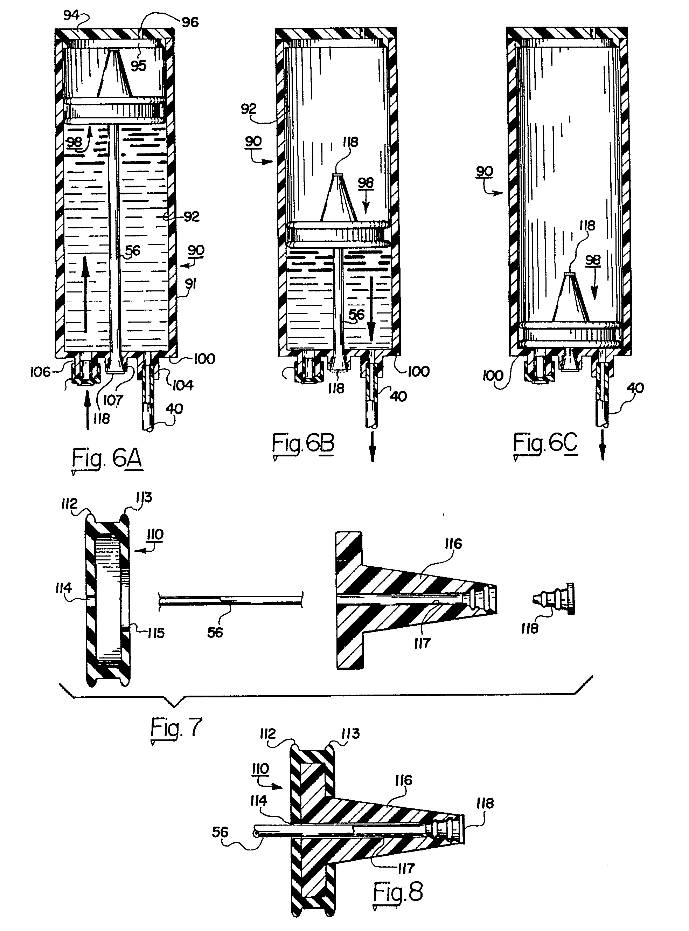

As depicted in Figs 7 and 8, the piston 98 has an outer member 110 made of Teflon (TM duPont), coated rubber or silicone rubber, or a surface treated or coated to prevent sticking or sliding friction. This member 110 is provided with ribs or rings 112 and 113, an aperture 114 in a forward wall and an enlarged aperture 115 in a rear wall. In addition, the piston 98 has a plastic molding 116 made to fit the inside of the outer member 110. This molding 116 has a through aperture 117 with a small tapered portion adapted to receive and retain a plug 118 when an elastomeric tubing 56 is brought into and through the aperture 117.

In Fig. 6 C, the piston 98 is shown adjacent the housing end wall 100. The tubing 40 is closed by the winged member 80 as in Fig. 5 above. With this tubing 40 closed, a needle is inserted through the plug 50 and fluid (drugs) is flowed into the housing 90 to cause the piston 98 to move rearwardly until the desired amount has been transferred. Tubing 56 is stretched to provide the desired constant tension as in Fig. 6 A. After the desired transfer (filling) has been achieved, connection to the patient is made and the piston 98, as moved by the stretched tubing 56, is caused to move toward end wall 100. The withdrawal of the needle causes the plug 50 to close and seal and, as shown in Fig. 6 B, the piston 98 is indicated as moving toward end wall 100. In Fig. 6 B, the piston 98 is shown midway, which is a position during filling and during the discharge of the fluid.

The assembly of the system or pump of FIGS. I through 5 anticipates high-speed, automatic or semi-automatic assembly of the several components identified above. The housing 20 is molded of an acceptable and inexpensive plastic, such as polypropylene, that is suitable for the drug or mixture to be used therein. The piston 28 is likewise made of materials suitable for short-term life. The sealing ribs or rings 31 and 32 on the added outer rim portion 30 are designed to provide a slideable seal for the period of intended use. If desired, the one-way valve ( not shown) or resilient plug 50 may be deleted in favor of a stopcock or rubber injection site provided in the tubing member.

The tubing 56 is to be an elastomeric rubber or stretchable rubber-like material and is to be retained by tapered plugs 58 (FIG. 2) or 118 (FIG. 7), which is a very positive securement means. A rubber band may also be utilized, but mechanical manupulation is more easily achieved with tubing which usually and more nearly provides a constant force through a given length of stretch. When the piston is adjacent the forward end of the housing, the tubing is stretched sufficiently to insure a constant force that will move the piston alongside the forward end of the housing.

In the embodiment of FIG. 6 A through FIG. 8, the front wall 100 is substantially flat so that the apertures 102, 104 and 106 are formed easily with and around pins extending from the mold core. As the cap 94 does not come in retentive contact with the fluid to the forward side of piston 98, this cap 94 may be of a different material from the housing 90. The housing 90 is contemplated to be of a transparent or substantially transparent material so that the user or patient may visually view the drug for clarity, contamination, etc., quantity infused or remaining, and readily determine flow rate. A flow rate is established by the aperture 76 provided in the luer lock connector 70 (FIG. 5). A winged cap 80 is conventionally provided, but other means, such as clamps, are known to shut off flow. The connector tubing may be of small bore to minimize fluid hold-up. The interior lumen may be fluted or otherwise contoured so as to prevent a seal-off due to accidental kinkage of the connector tubing.

It is contemplated that this metered delivery may also have a flow delivery connected to the tubing 40 by which an added medication, such as a pain inhibitor (killer) may be selectively delivered to the patient.

**Claims**

1. An ambulatory infusion pump including a housing (20, 90) having an inlet aperture (46, 106) for passage of a drug into said housing and an outlet aperture (42, 104) for passage of a received drug from said housing and a piston (28, 98) slidably mounted in said housing (20, 90) characterized in having an elastomeric member (56) secured between and to said housing (20, 90) and said piston (28, 98) to stretch in response to movement of said piston (28, 98) during filling of said housing (28, 90) with a drug through said inlet aperture (46, 106) and to impose a constant force on said piston (28, 98) after filling for expelling the drug through said outlet aperture (42, 194).

2. An ambulatory infusion pump as set forth in claim 1 further characterized in that said elastomeric member (56) is a tube.

3. An ambulatory infusion pump as set forth in claim 2 which is further characterized in having a first plug (58) securing one end of said tube (56) in an end wall (54, 100) of said housing (20, 90) and a second plug (58) securing an opposite end of said tube (56) in said piston (28, 98).

4. An ambulatory infusion pump as set forth in any preceding claim wherein said outlet aperture (42, 104) is a restrictor for controlling the rate of drug flow from said housing (20, 90).

5. An ambulatory infusion pump as set forth in any preceding claim wherein said housing (20) has a first projecting tubular portion (54) secured to said elastomeric member (56), a second projecting tubular portion (36) having a recess (38) for receiving a flexible tubing (40) and a plurality of holes (60) between said tubular portions communicating with the interior of said housing (20).

6. An ambulatory infusion pump as set forth in claim 5 wherein said inlet aperture (46) is disposed in said second tubular portion (36).

7. An ambulatory infusion pump as set forth in claim 6 further characterized in having a one-way valve (50) closing said inlet aperture (46).

8. An Ambulatory infusion pump as set forth in claim 5 further characterized in that outlet aperture (42) is disposed centrally in said second tubular portion (36).

9. An ambulatory infusion pump as set forth in claim 1 or 2, wherein said housing (90) has a wall (100) having said apertures (106, 104) therein and having one end of said elastomeric member (56) secured thereto.

10. An ambulatory infusion pump as set forth in claim 9 characterized in having a one-way valve (50) closing said inlet aperture (106).

Fig. 1

Fig. 4

Fig. 2

Fig. 3

Fig. 5

Fig. 6A

Fig. 6B

Fig. 6C

Fig. 7

Fig. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 677 444  (MERRILL)<br>* claim 2; figures 2,4,5 * | 1,9 | A 61 M    5/14 |
| | --- | | |
| A | US-A-4 430 079  (THILL et al.)<br>* claim 1; figure 1 * | 4 | |
| | ----- | | |

**TECHNICAL FIELDS SEARCHED (Int. Cl.4)**

A 61 M    5/00

The present search report has been drawn up for all claims

| Place of search | Date of completion of the search | Examiner |
|---|---|---|
| BERLIN | 03-08-1987 | PAPA E.R. |

EPO Form 1503 03.82